# NEUE EUROPÄISCHE PATENTSCHRIFT

(11) **EP 0 922 226 B2**
(45) Veröffentlichungstag und Bekanntmachung des Hinweises auf die Entscheidung über den Einspruch: **25.06.2008**
(45) Hinweis auf die Patenterteilung: 12.03.2003
(21) Anmeldenummer: 97918977.6
(22) Anmeldetag: 13.08.1997
(51) Int. Cl.: G01N 33/68

(54) **VERFAHREN ZUR ERFASSUNG DES STATUS EINES ORGANISMUS DURCH MESSUNG VON PEPTIDEN**
PROCESS FOR DETERMINING THE STATUS OF AN ORGANISM BY PEPTIDE MEASUREMENT
PROCEDE DE DETERMINATION DE L'ETAT D'UN ORGANISME PAR MESURE DES PEPTIDES

(30) Priorität: 13.08.1996 DE 19632521; 16.06.1997 DE 19725362
(43) Veröffentlichungstag der Anmeldung: 16.06.1999
(73) Patentinhaber: Digilab, Inc., Canton, MA 02021 (US)
(72) Erfinder: FORSSMANN, Wolf-Georg, D-30625 Hannover (DE); SCHULZ-KNAPPE, Peter, D-30625 Hannover (DE); SCHRADER, Michael, D-30625 Hannover (DE); OPITZ, Hans-Georg, D-30625 Hannover (DE)
(74) Vertreter: Schnappauf, Georg
(86) Internationale Anmeldenummer: PCT/EP1997/004396
(87) Internationale Veröffentlichungsnummer: WO 1998/007036

(56) Entgegenhaltungen:
- A. HERNANZ ET AL.: "Gastrointestinal peptide profile in children with celiac disease." JOURNAL OF PEDIATRIC GASTROENTEROLOGY, Bd. 6, Nr. 3, 1987, NEW YORK NY USA, Seiten 341-345, XP002050736
- M.J. STAQUET ET AL.: "Keratin polypeptide profile in psoriatic epidermis normalized by treatment with etretinate." ARCHIVES OF DERMATOLOGICAL RESEARCH, Bd. 275, Nr. 2, 1983, BERLIN FRG, Seiten 124-129, XP002050737
- C.R. JIM¹NEZ ET AL.: "Pattern changes of pituitary peptides in rat after salt-loading as detected by means of direct, semiquantitative mass spectrometric profiling." PROCEEDINGS OF THE NATIONAL ACADEMY OF SCIENCES USA, Bd. 94, Nr. 17, 1997, BETHESDA MD USA, Seiten 9481-9486, XP002050738
- W F Ganong, Lehrb der Med Physiologie (Springer 1979) 486-493
- K Lipp and H Wiegand (1973) Hoppe Zeylers Z Physiol Chem 354, 262-266
- G C Patrick et al (1980) Arch Pathol Lab Med 104, 293-299
- G H Fridland and D M Desiderio (1986) J Chromatography 379, 251-268
- K Yamashita et al (1993) J Biochem 114, 766-769
- E Gelpi (1995) J Chromatography A 703, 59-80

## Beschreibung

Gegenstand der vorliegenden Erfindung ist ein Verfahren zur Erfassung des Status eines Organismus durch Messung von Peptiden aus einer Probe des Organismus.

Zur Erfassung des Status eines Organismus werden verschiedene analytische Methoden eingesetzt. So wird beispielsweise in der Diagnostik von höheren Organismen bei pathologischen Befunden aufgrund der Symptomatik versucht, die Ursache der pathologischen Veränderung zu ergründen, um eine kausale Therapie zu entwickeln. Desweiteren ist man bemüht, durch Sequenzierung der Genome von Organismen und Etablierung von "Wildtyp-Genomen" eine Referenz eines durchschnittlichen, "gesunden" Organismus zu entwickeln, um dann individuelle Abweichungen, die auf mögliche pathogene Entwicklungen hinweisen können, durch entsprechende Genanalysen zu entdecken. Nachteilig an dem ersten methodischen Ansatz ist, daß man keine hypothesenfreie Diagnostik durchführen kann, da dabei eine Diagnose unternommen wird, die bereits auf Vermutungen beruht. Nachteilig an dem zweiten Verfahren ist, daß es auf lange Sicht noch nicht möglich sein wird, wichtige oder gar alle auf genetische Fehlfunktionen zurückzuführende Erkrankungen zu diagnostizieren. Ein weiterer Nachteil der zuletzt genannten Methode kann auch darin bestehen, daß eine Mutation auf einem Gen nicht unbedingt zur Expression des damit verbundenen Phänotypen führt.

Es wäre mithin wünschenswert, über ein universell einsetzbares diagnostisches Verfahren zu verfügen, mit welchem es gelingt, die geschilderten Nachteile zu vermeiden und insbesondere eine hypothesenfreie Diagnostik durchführen zu können. Das diagnostische Verfahren sollte darüber hinaus universell einsetzbar sein, nicht beschränkt bleiben auf höher entwickelte Systeme, sondern auch gleichfalls einsetzbar sein, um den Status von niederen Organismen zu erfassen. Es sollte darüber hinaus leicht etablierbar sein und mit an sich bekannten Techniken ausgeführt werden können.

Journal of Pediatric Gastroenterology, Bd. 6, Nr. 3, 1987, Seiten 341-345 beschreibt ein Verfahren zur Charakterisierung des Plasma-Profils von Gastrointestinai-Peptiden von an Celiac-Krankheit erkrankten Kindern im Vergleich mit dem gesunder Kinder. Archives of Dermatological Research, Bd. 275, Nr. 2, 1983, Seiten 124-129 beschreibt die unterschiedliche Expression von Epidermis-Keratinen in Psoriasispatienten im Vergleich zur Normal-Expression von Epidermis-Keratinen bei Gesunden.

Ein der Erfindung zugrundeliegendes technisches Problem liegt mithin in der Bereitstellung eines solchen Verfahrens.

Überraschenderweise wird das der Erfindung zugrundeliegende technische Problem in einfacher Weise durch ein Verfahren mit den Merkmalen des Anspruchs 1 gelöst. Die Unteransprüche betreffen bevorzugte Ausführungsformen des erfindungsgemäßen Verfahrens.

Das erfindungsgemäße Verfahren zur Erfassung des Status eines Organismus geht davon aus, daß dem zu untersuchenden Organismus eine Probe entnommen wird. Die Probe kann auch der vollständige Organismus sein. Die Probe muß neben niedrigmolekularen Peptiden auch hochmolekulare Peptide oder Proteine enthalten. Die niedrigmolekularen Peptide werden dabei erfindungsgemäß durch Massenspektrometrie direkt erfaßt und charakterisiert und dienen als Indikator für den Status des Organismus. Dabei werden alle in der Probe befindlichen und meßtechnisch erfaßbaren niedermolekularen Peptide erfasst. Anders als bei herkömmlichen analytischen oder diagnostischen Methoden, wie die Gel-Elektrophorese oder die zweidimensionale Elektrophorese und beispielsweise klinische diagnostische Methoden, werden hier nicht die hochmolekularen Strukturen, wie beispielsweise Proteine untersucht. Im Gegensatz zu an sich bekannten diagnostischen Methoden, wie beispielsweise Radioimmunassay oder anderen Kompetitionsassays zur Messung von Peptidhormonen und ähnlichem, werden erfindungsgemäß die niedermolekularen Peptide direkt meßtechnisch erfaßt und nicht wie in den genannten Methoden indirekt. Als Referenz dient die Verteilung niedrigmolekularer Peptide bei einem repräsentativen Querschnitt von definierten Kontrollen.

Im erfindungsgemäßen Verfahren kann die zu untersuchende Probe von Geweben- oder Flüssigkeitsproben aus dem Organismus, dessen Status aufgenommen werden soll, stammen oder es kann der Organismus selbst oder Teile davon sein. Im Falle der Untersuchung niederer Organismen dient vorzugsweise der Organismus selbst als Probe. Als niedere Organismen kommen insbesondere Einzeller, wie prokaryontische Systeme oder einfache eukaryontische Systeme, wie Hefen oder andere Mikroorganismen, in Betracht.

Erfindungsgemäß sollen die niedrigmolekularen Peptide, die zur Messung herangezogen werden, vorzugsweise ein Molekulargewicht von höchstens 30.000 Dalton aufweisen. Die untere Grenze ist an sich nicht kritisch, jedoch stellen Dipeptide die untere Grenze der niedrigmolekularen Peptide, die erfindungsgemäß erfaßt werden sollen, dar. Insbesondere bevorzugt sind Molekulargewichte der niedrigmolekularen Peptide von 100 bis 10.000 Dalton.

Falls erforderlich, weil beispielsweise durch eine veränderte Meßanordnung bedingt, kann es vorteilhaft sein, hochmolekulare Peptide oder Proteine sowie andere Biopolymere, die möglicherweise mit der Messung interferieren, aus der Probe zu entfernen. Dies ist insbesondere dann nicht erforderlich, wenn durch die erfindungsgemäß einzusetzende Meßmethode die höhermolekularen Peptidverbindungen meßtechnisch nicht erfaßt werden.

Insbesondere bewährt hat sich die sogenannte MALDI-Methode (Matrixunterstützte Laser-Desorptions-Ionisations-Massenspektroskopie). Es empfiehlt sich, die durch die Massenspektroskopie ermittelbaren Daten zur Charakterisierung der niedermolekularen Peptide einzusetzen, wie beispielsweise deren Molekulargewicht. Es ist ebenfalls möglich, unter bestimmten Umständen andere Parameter zu analysieren, wie beispielsweise die Ladung der Peptide oder die charakteristische Retentionszeit auf Chromatographiesäulen oder ein Fragmentmuster der niedermolekularen Peptide oder Kombinationen aus Masse der niedrigmolekularen Peptide und deren Ladungen.

Je nach Fragestellung, die mit der Erfassung des Status des Organismus noch verbunden ist, kann es vorteilhaft sein, die Probe auf mehrere Fraktionen zu verteilen und die Proben unter verschiedenen Fragestellungen oder meßtechnischen Anordnungen zu analysieren und somit einen Status des Organismus zu erfassen.

Als Organismen dienen insbesondere Prokaryonten, Eukaryonten, vielzellige Organismen, Zellen aus Gewebekulturen, Zellen von Tieren und Menschen. So wird es erfindungsgemäß ermöglicht, den Status von genetisch veränderten oder transformierten und/oder konditionierten Organismen zu untersuchen. Dies kann insbesondere bei Überprüfungen von transformierten Systemen vorteilhaft sein, um zu erkennen, inwieweit transformierte Organismen möglicherweise unerwartete oder unerwünschte Eigenschaften entwickelt haben, indem beispielsweise Peptide gebildet werden, die auf unerwünschte oder unerwartete Eigenschaften, wie toxische Eigenschaften, hinweisen.

Das erfindungsgemäße Verfahren ermöglicht mithin die Erfassung des Status eines transformierten Organismus mit hypothesefreier Untersuchung und Aufnahme des Status des Gesamtorganismus zur Aufdeckung von Veränderungen des transformierten Organismus, zur Aufdeckung von mit der Transformation verbundenem Auftreten von Peptiden, die kausal mit Stoffwechselveränderungen zusammenhängen.

Insbesondere kann jede bewußt oder unbewußt vorgenommene Manipulation (Konditionierung) eines Organismus dessen Status beeinflussen, sei es im Rahmen der Verabreichung von Medikamenten, der Gentherapie, bei Infektionen, am Arbeitsplatz durch Kontakt mit chemischen Stoffen, bei Versuchstieren, insbesondere transgenen Tieren und knock-out-Mutanten. Insbesondere bei solchen Verfahren kann durch den intra- und inter-individuellen Vergleich, beispielsweise durch chronologische Probenentnahme aus einem Organismus vor und im Verlauf einer der oben genannten Maßnahmen oder durch Vergleich mit nicht behandelten Kontrollorganismen, überprüft werden, ob die vorhergesagten, erwünschten Änderungen im Status tatsächlich eingetreten sind und ob darüberhinaus oder stattdessen nicht vorhergesagte, unerwünschte oder auch erwünschte Änderungen eingetreten sind, die durch das erfindungsgemäße Verfahren hypothesenfrei erfaßt werden.

Daher eignet sich das erfindungsgemäße Verfahren auch zum Beispiel zur Begleitung von klinischen Studien, toxikologischen Untersuchungen bei der Erprobung von Medikamenten aller Art, zur Analyse/Erfassung von Abbauprodukten, zur Identifikation von Genprodukten.

In der Veterinär- und Humanmedizin entwickelt das erfindungsgemäße Verfahren seine überragende Bedeutung dadurch, daß eine hypothesenfreie Erfassung des Status des betreffenden Organismus ermöglicht wird. Es wird also nicht bereits mit einer vorgefaßten Meinung ein Bestätigungsassay durchgeführt, sondern es wird ein echtes Gesamtbild des Status des untersuchten Organismus erstellbar. Das erfindungsgemäße Verfahren, daß als differentielles Peptiddisplay (Differential Peptide Display) bezeichnet werden kann, geht dabei davon aus, daß in einem gesunden Organismus ein bestimmtes Peptidmuster vorhanden ist und deshalb in der Lage ist, als Referenzstandard zu dienen. Nimmt man nun den Peptidstatus eines Individuums auf und vergleicht diesen mit der Referenz, so kann man einerseits Abweichungen feststellen, die bereits einen ersten Hinweis auf einen möglicherweise pathogenen Zustand geben. Werden nunmehr die Abweichungen, die durch Vergleich mit ähnlichen pathogenen Zuständen erstellt worden sind, aus entsprechenden Proben eines Erkrankten ermittelt, so kann bereits durch Vergleich der Abweichungen im Peptidmuster der Probe des Individuums und Übereinstimmung der Abweichung mit einem zugeordneten Krankheitsbild die betreffende Erkrankung direkt aus der Analyse identifiziert werden.

Erfindungsgemäß kann dabei insbesondere wie folgt vorgegangen werden. Zur Herstellung einer Referenzprobe können zunächst Ultrafiltrate aus Körperflüssigkeiten und Gewebsextrakten verwendet werden. Die Gewinnung der Filtratpeptide und ihre Auftrennung in Fraktionen erfolgt, indem beispielsweise niedrigmolekulare Peptidfraktionen gewonnen werden. Die Charakterisierung der Peptidfraktionen kann beispielsweise anhand von Retentionsverhalten und molekularer Masse, ermittelbar durch Chromatographie oder Massenspektroskopie, erfolgen. Wird beispielsweise Ultrafiltrat von Patienten, die an einer bekannten Erkrankung leiden, verwendet und dieses mit dem zuvor erstellten Spektrum von gesunden Referenzprobanden verglichen, kann durch das abweichende Muster eine Zuordnung der spezifischen Erkrankung mit dem Status des betreffenden Peptidgemisches erfolgen. Analysiert man eine Probe aus einem Patienten, bei dem ein bestimmtes Erkrankungsbild erkennbar ist und eine Hypothese für die Ursache dieser Erkrankung besteht, kann beispielsweise dieses spezifische Peptid in der Analyse ebenfalls abgefragt werden und bei positivem Ausgang entsprechende Therapiepläne eingerichtet werden. So ist es durchaus möglich, zunächst dem Patienten eine Probe zu entnehmen, mit dem erfindungsgemäßen Verfahren einen Status aufzunehmen, um dann bei Feststellen des Vorliegens einer auf pathogene Zustände hinweisenden Abweichung entweder durch an sich bekannte Bestätigungsassays, unter Heranziehung der üblichen klinischen Assays, eine Kontrollmessung durchzuführen oder die Kontrollmessung durch spezifisches Screening nach dem Indikator des pathogenen Zustands durchzuführen.

Peptide können dabei nach dem Fachmann bekannten Verfahren, wie beispielsweise Ultraf ltration des entsprechenden Ausgangsmaterials, gewonnen werden. Dabei werden Filter mit einer molekularen Ausschlußgröße verwendet, die in dem erfindungsgemäß beanspruchten Bereich liegen, also zwischen denen eines Dipeptides und maximal 30.000 Dalton. Durch geeignete Wahl der jeweiligen Membranen können auch bestimmte Molekulargewichtsfraktionen gewonnen werden. Vorzugsweise werden im Rahmen der Filtration 0,2 ml bis 50 l Filtrat gewonnen, das beispielsweise sofort nach Abschluß der Filtration durch Ansäuern mit verdünnter Salzsäure auf einen pH-Wert von 2 bis 4 eingestellt wird. Die genannten Mengen dienen insbesondere dazu, gepoolte Proben zu untersuchen, zum einen zur Entwicklung von Referenzproben gesunder Probanden bzw. zur Bestimmung krankheitsspezifischer Peptidmarker zur Erstellung einer Peptiddatenbank.

Die nach Ultrafiltration im Filtrat vorliegenden Peptide werden durch Adsorption an chromatographische Materialien, insbesondere Kationenaustauscher, wie beispielsweise Fractogel, Anionenaustauscher-Fractogel TMAE und Reverse-Phase-(RP)-Materialien, mit nachfolgender Elution durch lineare Gradienten oder Stufengradienten gewonnen. Zur weiteren Aufreinigung können gegebenenfalls weitere chromatographische Trennungen, insbesondere über RP-Phasenmaterial durchgeführt werden.

Die Erfassung der Peptidfraktionen erfolgt durch massenspektrometrische Analyse, insbesondere mit der MALDI-MS (matrix assisted laser desorption ionisation mass spectrometry) oder ESI-MS (electro spray ionisation-MS). Dies sind Methoden, die zur Analyse von Peptiden einsetzbar sind. Hierbei wird vorzugsweise mit einer On-Line-Kopplung einer Microbore RP-Trennung und der Massenspektrometrie (LC-MS-Kopplung) gearbeitet. Aus den erhaltenen Daten wird eine mehrdimensionale Tabelle nach Retentionsverhalten, Molekulargewicht und Signalintensität als bevorzugte Leitparameter erstellt. Es können jedoch auch andere mit den genannten Methoden ermittelbare Größen erfaßt werden.

Die über die vorgenannten Schritte gewonnenen Daten über Patienten mit einer bekannten Grunderkrankung werden mit den gleichartig gewonnenen Daten einer gesunden Referenzpopulation verglichen. Hierbei werden sowohl qualitative Änderungen (z. B. das Auftreten neuer Peptide oder das Fehlen von Peptiden), als auch quantitative Änderungen (das vermehrte beziehungsweise verminderte Auftreten von einzelnen Peptiden) festgestellt. Die über die vergleichende Analyse definierten Targets können, falls erforderlich, im weiteren durch den Fachmann bekannte Methoden peptidchemisch gereinigt und identifiziert werden. Die erhaltenen Sequenzinformationen können dann mit Protein- und Nucleinsäuredatenbanken sowie nachfolgend mit Literaturdaten verglichen werden. Die Relevanz der dargestellten Peptide bezüglich der untersuchten Erkrankung wird überprüft durch funktionelle Studien und durch Reihenscreening an geeigneten Patientengruppen.

### Beispiel 1

Verwendung von Körperflüssigkeiten, hier: Blutfiltrat (Hämofiltrat, HF)

### 1. Gewinnung von HF

HF wird im Rahmen einer arterio-venösen oder auch venovenösen Hämofiltration nach dem Fachmann bekannten Techniken an ausgewählten Patienten oder Probanden durchgeführt. Die Gewinnung von HF erfolgt in der Weise, wie sie im Prinzip bei chronisch nierenkranken Patienten routinemäßig durchgeführt wird. Über eine arterielle Ableitung und venöse Zuleitung (arterio-venöse HF) oder eine venöse Ableitung mit venöser Zuleitung (veno-venöse HF) wird das Blut des Patienten unter apparativer Unterstützung durch ein Hämofiltratsgerät (z. B. Hemoprozessor, Sartorius, Göttingen; AK 10 HFM, Gambro, Hechingen) über ein Hämofilter geleitet (z. B. Hemoflow F 60 oder Hemoflow HF 80 S, Fresenius, Bad Homburg; Hemoflow FH 77 H und Hemoflow HF 88 H, Gambro), das eine molekulare Ausschlußgröße von bis zu 30 kDa besitzt. Das dem Patienten entzogene Filtratvolumen wird durch eine Elektrolytlösung substituiert (z. B. SH 01, SH 05, SH 22, SH29, Schiwa, Glandorf).

Im Rahmen des hier vorliegenden Verfahrens wird eine diagnostische Hämofiltration mit dem Ziel durchgeführt, zwischen 1 und 30 l HF bei einem Patienten innerhalb einer Hämofiltration zu gewinnen. Das Hämofiltrat wird zur Vermeidung der Proteolyse sofort mit verdünnter Säure (z. B. 1 M HCl) auf einen pH-Wert zwischen 2 und 4 eingestellt und auf 4°C gekühlt.

### 2. Gewinnung der HF-Peptide und Auftrennung in Fraktionen

### 2.1 Peptidextraktion mit stufenweiser Elution

10 l Hämofiltrat werden mit entionisiertem Wasser auf eine Leitfähigkeit von 6 mS/cm verdünnt und der pH mit Salzsäure auf 2,7 eingestellt. Das HF wird dann auf eine Chromatographiesäule aufgetragen. Nach Bindung der HF-Peptide werden die gebundenen Peptide mit einer pH-Stufenelution eluiert. Dabei werden 7 Puffer mit aufsteigendem pH verwendet.

Chromatographiebedingungen:
Fluß beim Auftrag: 100 ml/min
Fluß beim Eluieren: 30 ml/min
Detektion: 214, 280 nm
Säule: Vantage (Amicon, Witten) 6 cm Durchmesser x 7 cm Füllhöhe
Säulenmaterial: Fraktogel TSK SP 650 M (Merck, Darmstadt)
Anlage: BioCAD 250, Perseptive Biosystems, Wiesbaden-Nordenstadt

| Puffer | pH-Wert | Puffersubstanzen | Molarität |
|---|---|---|---|
| Elutionspuffer 1 | 3,6 | Zitronensäure | 0,1 |
| Elutionspuffer 2 | 4,5 | Essigsäure | 0,1 |
| Elutionspuffer 3 | 5,0 | Apfelsäure | 0,1 |
| Elutionspuffer 4 | 5,6 | Bernsteinsäure | 0,1 |
| Elutionspuffer 5 | 6,6 | Natriumdihydrogenphosphat | 0,1 |
| Elutionspuffer 6 | 7,4 | Dinatriumhydrogenphosphat | 0,1 |
| Elutionspuffer 7 | 9,0 | Ammoniumcarbonat | 0,1 |

| | | | |
|---|---|---|---|
| Die Eluate 1 - 7 werden separat gesammelt. | | | |

### 2.2 Zweite chromatographische Auftrennung

Die Eluate 1 - 7 werden separat über eine Reverse-Phase-Säule chromatographiert.

Chromatographiebedingungen:
Fluß beim Auftrag: 10 ml/min
Fluß beim Eluieren: 4 ml/min
Detektion: 214 nm
Säule: HPLC-Stahlsäure, 1 cm Durchmesser, 12,5 Füllhöhe
Säulenmaterial: Source RPC 15 µm (Pharmacia, Freiburg)
Anlage: BioCAD, Perseptive Biosystems, Wiesbaden-Nordenstadt

Das Eluat wird in 4 ml-Fraktionen gesammelt.

### 3. Kartierung der Peptid-Fraktionen

### 3.1

Aliquots der in 2.2 gewonnen Fraktionen werden auf einer Microbore-Reverse-Phase-Säule aufgetragen und im Gradient eluiert. Die Detektion erfolgt mit UV-Detektor und on-line mit einem Elektrospray-Massenspektrometer.

Chromatographiebedingungen:
Fluß beim Auftragen: 20 µl/min
Fluß beim Eluieren: 20 µl/min
Detektion: 220 nm
Säule: C18 AQS, 3 µm, 120 A, 1 mm Durchmesser, 10 cm Länge
(YMC, Schermbeck)
Anlage: ABI 140 B Dual solvent Delivery System

Puffer A: 0,06% Trifluoressigsäure in Wasser
Puffer B: 80% Acetonitril in A
Gradient: 0% B auf 100% B in 90 min

On-Line-Massenspektrometrie:
API III mit Elektrospray-Interface (Perkin-Elmer, Weiterstadt)
Positive Ion Modus
Meßbereich: m/z von 300 bis 2.390
Scan-Zeit: 7 sec
Scan-Fenster: 0,25 m/z

Datenerfassung erfolgt mit MacSpec oder MultiView Software (Perkin-Elmer).

### 3.2 MALDI-MS Messung der einzelnen Fraktionen

Aliquots der in 2.2 gewonnen Fraktionen werden mit unterschiedlichen Matrixsubstanzen, z. B. unter Zusatz von L(-) Fucose im MALDI-MS gemessen.

Aus den Rohdaten wird eine mehrdimensionale Tabelle erstellt unter Berücksichtigung der Scan-Nummer, Signalintensität und nach Kalkulation der Massen aus den multipel geladenen Ionen eines Scans.

### 4. Vergleichende Analyse

### 4.1 Identifikation neuer, fehlender oder in ihrer Menge deutlich verschiedener Peptide

Durch Vergleich der unter 3.3 erhaltenen Datensätze, die auch als Peptidkarten bezeichnet werden können, werden qualitative und/oder quantitative Unterschiede festgestellt. Dabei werden unter Berücksichtigung von Kontrollen und Proben einzelne Datensätze oder auch Gruppen von Datensätzen zum Vergleich herangezogen.

### 4.2 Peptidchemische Charakterisierung der identifizierten Targets

Aus dem gewonnen Rohmaterial (z. B. Großpräparationen von Hämofiltrat) werden die identifizierten Targets in Mengen aufgereinigt, die eine Identifikation erlauben. Dazu werden die unterschiedlichen, dem Fachmann bekannten chromatographischen Trenntechniken (Reverse Phase, Ionenaustausch, Ausschlußgrößenchromatographie, hydrophobe Interaktions-Chromatographie etc.), die im allgemeinen zur Auftrennung von Peptidgemischen eingesetzt werden, verwendet. Nach jeder chromatographischen Trennung einer Fraktion werden über ESI-MS, MALDI-MS oder auch LC-MS die Targets erneut in den Fraktionen identifiziert. Dieses Procedere wird unter Variation der chromatographischen Parameter so oft wiederholt, bis ein reines Produkt der gesuchten Spezifikation, d. h. Retentionszeit und molekularer Masse, vorliegt. Darauf folgt die Bestimmung einer Teil- oder Komplett-Aminosäuresequenz oder eines Fragmentmusters. Im Anschluß wird ein Datenbankvergleich durchgeführt an den bekannten Datenbanken (Swiss-Prot und EMBL-Peptid- und Nucleinsäure-Datenbank) mit dem Ziel der Identifikation der Teil- oder Komplettsequenz oder eines Fragmentmusters. Ist kein Datenbankeintrag vorhanden, erfolgt die Aufklärung der Primärstruktur.

### Beispiel 2:

Verwendung von Körperflüssigkeiten, hier: Aszites

### 1. Gewinnung von Aszites

Aszites bildet sich als extravasales Exsudat bei unterschiedlichen Erkrankungen (maligne Tumoren, Leberstörungen etc.). Im Rahmen des hier vorliegenden Verfahrens werden zwischen 10 ml und 10 l Aszites durch Punktion gewonnen und danach zur Vermeidung der Proteolyse sofort mit verdünnter Säure (z. B. 1 M HCl) auf einen pH-Wert zwischen 2,0 und 4,0 eingestellt und auf 4°C gekühlt. Nach einer Ultrafiltration über eine Cellulose-Triacetat-Membran mit einer Ausschlußgröße von 30 kDa (Sartocon-Mini-Apparatur, Sartorius) wird das Filtrat als Quelle von Peptiden im weiteren verwendet.

### 2. Gewinnung der Aszites-Peptide und Auftrennung in Fraktionen

### 2.1. Peptidextraktion mit Gradienten-Elution

5 l Aszites-Filtrat werden auf pH 2,0 eingestellt und über eine präparative Reverse-Phase-Säule getrennt.

Chromatographiebedingungen:
Fluß beim Auftrag 40 ml/min
Fluß beim Eluieren: 40 ml/min
Detektion: 214 nm, 280 nm
Säule: Waters Kartuschensystem, 4,7 cm Durchmesser, 30 cm Füllhöhe
Säulenmaterial: Vydac RP-C18, 15 - 20 µm
Anlage: BioCAD, Perseptive Biosystems, Wiesbaden-Nordenstadt Puffer A: 0,1% Trifluoressigsäure in Wasser
Puffer B: 80% Acetonitril in A
Gradient: 0% B auf 100% B in 3,000 ml

Das Eluat wird in 50 ml Fraktionen gesammelt.

Der weitere Verlauf der Charakterisierung entspricht Beispiel 1.

### Beispiel 3:

### Verwendung von Körperflüssigkeiten, hier: Urin

### 1. Gewinnung von Urin

Urin wird direkt als Katheterurin oder als Spontanurin von Patienten in Mengen von 0,5 bis 50 l gewonnen und zur Vermeidung der Proteolyse sofort mit verdünnter Säure (z. B. 1 M HCl) auf einen pH-Wert zwischen 2,0 und 4,0 eingestellt und auf 4°C gekühlt. Nach einer Ultrafiltration über eine Cellulose-Triacetat-Membran mit einer Ausschlußgröße von 30 kDa (Sartocon-Mini-Apparatur, Sartorius) wird das Filtrat als Quelle von Peptiden im weiteren verwendet.

### 2. Gewinnung der Urin-Peptide und Auftrennung in Fraktionen

### 2.1 Peptidextraktion mit stufenweiser Elution

10 l Urin-Filtrat werden mit Wasser auf eine Leitfähigkeit von 6 mS/cm verdünnt und der pH mit HCl auf 2,7 eingestellt. Das Urin-Filtrat wird dann auf eine Chromatographiesäule aufgetragen. Nach Bindung der Peptide werden die gebundenen Peptide mit einem Kochsalzgradienten eluiert.

Chromatographiebedingungen:
Fluß beim Auftrag: 100 ml/min
Fluß beim Eluieren: 30 ml/min
Detektion: 214 nm
Säule: Vantage (Amicon, Witten) 6 cm Durchmesser x 7 cm Füllhöhe
Säulenmaterial: Merck Fraktogel TSK SP 650 M
Anlage: BioCAD 250, Perseptive Biosystems, Wiesbaden-Nordenstadt

Puffer A: 50 mM NaH₂PO₄ pH 3, 0
Puffer B: 1,5 M NaCl in A
Gradient: 0% B auf 100% B in 2,000 ml

Das Eluat wird in 10 Pools á 200 ml gesammelt.

### 2.2 Zweite chromatographische Auftrennung

Die Fraktionen werden separat über eine Reverse-Phase-Säule chromatographiert.

Chromatographiebedingungen:
Fluß beim Auftrag: 10 ml/min
Fluß beim Eluieren: 4 ml/min
Detektion: 214 nm
Säule: HPLC-Stahlsäule, 1 cm Durchmesser, 12,5 cm Füllhöhe Säulenmaterial: Pharmacia Source RPC 15 µm
Anlage: BioCAD, Perseptive Biosystems, Wiesbaden-Nordenstadt

Puffer A: 0,1% Trifluoressigsäure in Wasser
Puffer B: 80% Acetonitril in A
Gradient: 0% B auf 100% B in 200 ml

Das Eluat wird in 4ml Fraktionen gesammelt.

Der weitere Verlauf der Charakterisierung entspricht Beispiel 1.

## Patentansprüche

1. Verfahren zur Erfassung des Status eines Organismus durch hypothesefreie Messung eines Peptidmusters einer Probe des Organismus, die hoch- und niedrigmolekulare Peptide enthält, als Indikator für den Status des Organismus, wobei
- alle in der Probe befindlichen und meßtechnisch erfaßbaren niedrigmolekularen Peptide durch Massenspektrometrie direkt erfaßt und charakterisiert und
- mit einer Referenz in Beziehung gesetzt werden.

2. Verfahren nach Anspruch 1, wobei die Probe Gewebe- oder Flüssigkeitsproben aus dem Organismus oder, im Falle der Untersuchung niederer Organismen, der Organismus selbst oder Kombinationen davon ist.

3. Verfahren nach Anspruch 1 und/oder 2, wobei die niedrigmolekularen Peptide, die zur Messung herangezogen werden, ein Molekulargewicht von höchstens 30 000 Dalton aufweisen.

4. Verfahren nach Anspruch 3, wobei die niedrigmolekularen Peptide, die zur Messung herangezogen werden, mindestens ein Molekulargewicht, das dem von Dipeptiden entspricht, aufweisen.

5. Verfahren nach Anspruch 3 und/oder 4, wobei die niedrigmolekularen Peptide, die zur Messung herangezogen werden, ein Molekulargewicht von 100 bis 10 000 Dalton aufweisen.

6. Verfahren nach mindestens einem der Ansprüche 1 bis 5, wobei die hochmolekularen Peptide vor der Messung der niedrigmolekularen Peptide abgetrennt werden oder meßtechnisch oder auswertetechnisch bei der Erfassung der Probe nicht berücksichtigt werden.

7. Verfahren nach mindestens einem der Ansprüche 1 bis 6, wobei die niedrigmolekularen Peptide durch die Messung ihres Molekulargewichtes charakterisiert werden.

8. Verfahren nach mindestens einem der Ansprüche 1 bis 7, wobei die Probe vor der Messung der niedrigmolekularen Peptide in verschiedene Fraktionen aufgeteilt wird und unter unterschiedlichen Bedingungen gemessen wird.

9. Verfahren nach mindestens einem der Ansprüche 1 bis 8, wobei als Organismus Prokaryonten, Eukaryonten, vielzellige Organismen, Zellen aus Gewebekulturen, Zellen aus Tieren und Menschen dienen.

10. Verfahren nach mindestens einem der Ansprüche 1 bis 9, wobei die Probe aus genetisch veränderten oder transformierten und/oder konditionierten Organismen stammt.

## Claims

1. A method for detecting the condition of an organism through the measurement of a peptide pattern of a sample of said organism containing high- and low-molecular weight peptides, as an indication of the condition of said organism, without the need to recur to hypotheses, wherein
- all low-molecular weight peptides present in the sample which can be detected by the measurement technique are directly detected by mass spectroscopy and **characterized**; and
- related to a reference.

2. The method according to claim 1, wherein said sample is tissue or fluid samples from said organism, or the organism itself in the case of examining lower organisms, or combinations thereof.

3. The method according to claims 1 and/or 2, wherein said low-molecular weight peptides used for said measurement have a molecular weight of not more than 30,000 Dalton.

4. The method according to claim 3, wherein said low-molecular weight peptides used for said measurement have a molecular weight which at least corresponds to that of dipeptides.

5. The method according to claims 3 and/or 4, wherein said low-molecular weight peptides used for said measurement have a molecular weight of from 100 to 10,000 Dalton.

6. The method according to at least one of claims 1 to 5, wherein said high-molecular weight peptides are separated off prior to measurement of said low-molecular weight peptides, or left unconsidered, in terms of measurement or evaluation, in the recording of the sample.

7. The method according to at least one of claims 1 to 6, wherein said low-molecular weight peptides are **characterized** through the measurement of their molecular weights.

8. The method according to at least one of claims 1 to 7, wherein said sample is divided into different fractions prior to said measurement of the low-molecular weight peptides, and the fractions are measured under different conditions.

9. The method according to at least one of claims 1 to 8, wherein said organisms include prokaryotes, eukaryotes, multicellular organisms, cells from tissue cultures, cells from animals and humans.

10. The method according to at least one of claims 1 to 9, wherein said sample is derived from genetically engineered or transformed and/or conditioned organisms.

## Revendications

1. Procédé de détection de l'état d'un organisme par mesure non hypothétique d'un profil peptidique provenant d'un échantillon de l'organisme, qui contient des peptides de poids moléculaire élevé et des peptides de poids moléculaire bas, en tant qu'indicateur de l'état de l'organisme, dans lequel
- tous les peptides de poids moléculaire bas se trouvant dans l'échantillon et détectables sont détectés et **caractérisés** directement par spectrométrie de masse et
- sont mis en relation par rapport à une référence.

2. Procédé selon la revendication 1, dans lequel l'échantillon est constitué d'échantillons de tissu ou de liquide de l'organisme ou, dans le cas de l'analyse d'organismes inférieurs, de l'organisme lui-même, ou de combinaisons de ceux-ci.

3. Procédé selon la revendication 1 et/ou 2, dans lequel les peptides de poids moléculaire bas qu'on utilise pour la mesure présentent un poids moléculaire de 30 000 daltons au plus.

4. Procédé selon la revendication 3, dans lequel les peptides de poids moléculaire bas qu'on utilise pour la mesure présentent un poids moléculaire qui correspond à celui des dipeptides.

5. Procédé selon la revendication 3 et/ou 4, dans lequel les peptides de poids moléculaire bas qu'on utilise pour la mesure présentent un poids moléculaire de 100 à 10 000 daltons.

6. Procédé selon au moins l'une des revendications 1 à 5, dans lequel les peptides de poids moléculaire élevé sont séparés des peptides de poids moléculaire bas avant la mesure ou ne sont pas pris en considération du point de vue de la technique de mesure ou du point de vue de la technique d'évaluation lors de la détection de l'échantillon.

7. Procédé selon au moins l'une des revendications 1 à 6, dans lequel on caractérise les peptides de poids moléculaire bas par mesure de leur poids moléculaire.

8. Procédé selon au moins l'une des revendications 1 à 7, dans lequel l'échantillon est réparti en diverses fractions avant la mesure des peptides de poids moléculaire bas et est mesuré dans des conditions diverses.

9. Procédé selon au moins l'une des revendications 1 à 8, dans lequel des procaryotes, des eucaryotes, des organismes pluricellulaires, des cellules provenant de cultures de tissu, des cellules d'animaux et d'êtres humains servent d'organisme.

10. Procédé selon au moins l'une des revendications 1 à 9, dans lequel l'échantillon provient d'organismes génétiquement modifiés ou transformés et/ou conditionnés.
